(19) Europäisches Patentamt
European Patent Office
Office européen des brevets

(11) EP 3 672 652 B1

(12) **EUROPEAN PATENT SPECIFICATION**

(45) Date of publication and mention
of the grant of the patent:
**26.01.2022 Bulletin 2022/04**

(21) Application number: **18755482.9**

(22) Date of filing: **22.08.2018**

(51) International Patent Classification (IPC):
*A61L 27/38* (2006.01)    *A61L 27/22* (2006.01)
*A61L 27/52* (2006.01)    *C12M 3/00* (2006.01)
*C12M 3/06* (2006.01)    *C12N 5/077* (2010.01)

(52) Cooperative Patent Classification (CPC):
**A61L 27/225; A61L 27/3817; A61L 27/3852;**
**A61L 27/52; C12N 5/0655;** A61L 2300/64;
A61L 2430/06; C12N 2513/00; C12N 2533/56

(86) International application number:
**PCT/EP2018/072658**

(87) International publication number:
**WO 2019/038322 (28.02.2019 Gazette 2019/09)**

(54) **ARTIFICIAL CARTILAGE AND METHOD FOR ITS PRODUCTION**

KÜNSTLICHER KNORPEL UND VERFAHREN ZU DESSEN HERSTELLUNG

STRUCTURE SEMI-CONDUCTRICE ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **22.08.2017 EP 17187267**

(43) Date of publication of application:
**01.07.2020 Bulletin 2020/27**

(60) Divisional application:
**21215074.2**

(73) Proprietors:
• **Veterinärmedizinische Universität Wien**
**1210 Vienna (AT)**
• **Technische Universität Wien**
**1040 Wien (AT)**

(72) Inventors:
• **JENNER, Florien**
**1210 Vienna (AT)**
• **ROSSER, Julie**
**1210 Vienna (AT)**
• **ERTL, Peter**
**1060 Vienna (AT)**

(74) Representative: **SONN Patentanwälte OG**
**Riemergasse 14**
**1010 Wien (AT)**

(56) References cited:
**WO-A1-2016/174607      WO-A2-2006/017176**
**WO-A2-2010/101708      US-A1- 2003 040 113**

• **DRAGO STICKER ET AL: "Multi-layered,**
**membrane-integrated microfluidics based on**
**replica molding of a thiol-ene epoxy thermoset**
**for organ-on-a-chip applications", LAB ON A**
**CHIP, vol. 15, no. 24, 1 January 2015 (2015-01-01),**
**pages 4542-4554, XP055452655, ISSN: 1473-0197,**
**DOI: 10.1039/C5LC01028D cited in the application**
• **PETER ERTL: "Recent Advances of Biologically**
**Inspired 3D Microfluidic Hydrogel Cell Culture**
**Systems", CELL BIOLOGY & CELL**
**METABOLISM, vol. 2, no. 1, 20 November 2015**
**(2015-11-20), pages 1-14, XP055452653, DOI:**
**10.24966/CBCM-1943/100005 cited in the**
**application**

- **FEDERICO TORTELLI ET AL: "THREE-DIMENSIONAL CULTURES OF OSTEOGENIC AND CHONDROGENIC CELLS: A TISSUE ENGINEERING APPROACH TO MIMIC BONE AND CARTILAGE IN VITRO", EUROPEAN CELLS AND MATERIALS, vol. 17, 1 January 2009 (2009-01-01), pages 1-14, XP055035014, ISSN: 1473-2262, DOI: 10.22203/eCM.v017a01 cited in the application**

Remarks:
The file contains technical information submitted after the application was filed and not included in this specification

**Description**

The present invention relates to three-dimensional chondrocyte cultures and uses thereof (e.g. as artificial cartilage or cartilage replacement), as well as methods for their production.

Osteoarthritis (OA) is a degenerative joint disease with intermittent inflammatory episodes. The disease can be induced by a single severe trauma, repetitive microtrauma, or strenuous exercise, and causes pain and decreased range of motion. OA affects an estimated 22.7% (52.5 million) of adults (> 18 years) in the United States. A study performed in 8 European countries showed that between 5 and 11% of the population aged 25-79 suffer from OA (Dalstra et al., Int. J. Epidemiol. 34 (2005), 316-326). OA has a major impact on human activity and is a considerable socioeconomic burden in terms of lost time at work and early retirement.

Articular cartilage is comprised of a single resident cell population, chondrocytes, and the specialized extracellular matrix (ECM) they produce. It provides a stable, smooth, wear-resistant, almost friction-free gliding surface and lends the ability to withstand high compressive and shear forces. Unfortunately, articular cartilage shows only minimal regeneration potential because there is a limited response of cartilage to tissue damage and an inadequate natural repair response from adjacent tissues. The natural repair processes seen in osteochondral lesions in adults result in fibrocartilaginous scar tissue. This repair tissue has inferior mechanical properties and therefore degenerates over time, precipitating intermittent joint inflammation and resulting in chronic OA. Cartilage repair strategies aim to resurface lesions and restore joint functionality. However, no repair method has yet been developed which consistently resembles native articular cartilage (Gelse et al., Osteoarthr. Cart. 20 (2012), 162-171). Current treatments include a wide range of non-pharmacological, pharmacological, and surgical modalities. The prognosis for patients suffering from OA is still poor since there are no effective pharmacological therapies available that alter the pathobiologic course of the disease (Felson et al., Ann. Int. Med. 133 (2000), 726-737).

Research into cartilage regeneration has traditionally been performed in monolayer cultures, creating a two-dimensional (2D) cellular environment in which cells adhere to a plastic surface with no matrix interaction. In monolayer, chondrocytes lose their articular phenotype and alter gene expression, favouring collagen I over the more specific hyaline component, collagen II. This process is described as "de-differentiation". Dedifferentiated chondrocytes exhibit a fibroblast-like cellular morphology. In contrast, three-dimensional (3D) cell culture simulates the native cellular environment, lending cells a physiologic atmosphere and bringing protein transcriptomics, secretome analyses, and gene expression to a level more closely resembling in vivo than ever before. Chondrocytes cultured in 3D display more physiologic, rounded cell morphology, described as "re-differentiated" (Capito et al., Osteoarthr. Cart. 14 (2006), 1203-1213).

3D cell culture is particularly interesting for investigation of cell lines normally situated within a dependent, functional ECM, such as chondrocytes. Cartilage ECM can be mimicked with biocompatible hydrogels, such as fibrin, with variable porosity and mechanical stiffness. 3D cultured chondrocytes display a more native morphology and secrete ECM components. Recently, a validated 3D in vitro model of osteoarthritis was established (Sun et al., Biomaterials 32 (2011), 5581-5589), where chondrocytes could be injured with the addition of cytokines tumor necrosis factor (TNF)-alpha and interleukin (IL)-1beta, or alternatively with the addition of macrophage conditioned medium. Bachmann et al. (Students and Young Investigators in Regenerative Medicine Scientific Forum, Danube University Krems, 1 April 2016 (abstract)) and Rosser et al. (International Cartilage Research Society 2016, Sorrento, Italy, 24-27 September 2016 (electronic poster)) discuss various general aspects of 3D in-vitro osteoarthritis models.

Furthermore, animal trials inevitably include additional unquantified variables, such as animal health status, reproducible growth, diet, weight, and intrinsic genetic differences (Ertl et al., Europ. Pharm. Cont. (2009), 52-54; Sun et al., 2011). However, cell-based assays can be considered more reliable (Ertl et al., 2009) than animal trials for high throughput and drug testing by providing physiologically relevant three-dimensional cellular phenotypes and mimicking in vivo conditions.

Tortelli et. al. (European Cells and Materials 17 (2009): 1-14) relates to a tissue engineering approach to mimic bone and cartilage in vitro. It is stated that the design of an ideal model of cartilage is still a hard challenge in the field of tissue engineering (page 5, right column, second paragraph).

It is an object of the present invention to provide improved chondrocyte cultures which more closely resemble the natural cartilage. Such cultures should have the main three-dimensional characteristics of cartilage, specifically with respect to chondrocyte activity and metabolism. The cultures may then be used in various OA models and to test substances for OA therapy, as well as in personalised medicine and surgery. The cultures should also be compatible with other components necessary for such models and test systems, e.g. with bioreactors, chips, etc.

The scope of the present invention is defined by the claims. Any "embodiment" or "example" which is disclosed in the description but is not covered by the claims should be considered as presented for illustrative purpose only.

The present invention provides a three-dimensional tissue culture, comprising chondrocytes in a biocompatible artificial matrix, as disclosed in present claim 7.

According to a particular preference, the culture further comprises a third layer at least partially covered by the second layer, wherein chondrocytes are arranged into columns extending into the matrix, wherein each column has at least two chondrocytes.

The present invention also provides a device, such as a microfluidic device, comprising the three-dimensional tissue culture of the present invention. This device can be used as a cartilage injury model, especially as an OA model.

The present invention provides a method for manufacturing a three-dimensional tissue culture comprising chondrocytes in a biocompatible artificial matrix, the method comprising the steps of:

- providing chondrocytes;

- dispersing the chondrocytes in an aqueous solution, wherein the solution comprises polymerisable molecules, such that an essentially homogenous dispersion is obtained;

- transferring at least a part of the dispersion into a casting mould;

- exposing the dispersion in the casting mould to conditions which allow polymerisation of the polymerisable molecules to obtain a matrix in which chondrocytes are present; wherein the matrix has a bovine serum albumin (BSA) diffusion coefficient of $2.5 \times 10^{-11}$ cm$^2$/s to $1 \times 10^{-6}$ cm$^2$/s at a temperature of 20°C; and

- culturing the chondrocytes in the matrix under growth conditions, wherein a portion of the surface of the matrix is in contact with a growth medium,

wherein said casting mould is a cell chamber of a microfluidic chip and said growth medium is brought in contact with said portion of the surface of the matrix through a medium channel of the microfluidic chip during the culturing.

The above method has turned out to be particularly suitable to induce formation of the first and the second layer in the culture.

The present invention further relates to a method for manufacturing a three-dimensional tissue culture comprising chondrocytes in a biocompatible artificial matrix, wherein the casting mould has a bulge, wherein the matrix least partially extends into the bulge; and wherein at least a portion of the matrix bulge is above the level of the growth medium.

The above method has turned out to be especially suitable to induce formation of the third layer in the culture, in addition to the first and the second layer.

The present invention also relates to a three-dimensional tissue culture obtainable by the inventive method, a device comprising such a culture and the use of such a device as a cartilage injury model, especially as an OA model.

The culture according to the present invention not only shows high cell viability but also metabolic activity and protein expression similar to native cartilage. Surprisingly, the chondrocytes obtained by the inventive method spontaneously align themselves in a structural organization similar to native cartilage, where chondrocytes establish a compacted pericellular environment (PCM) to form the primary structural, functional and metabolic unit of cartilage called chondron.

It is especially surprising that a layer formation similar to that of natural cartilage could be achieved in the course of the present invention, with the first layer being akin to the superficial zone of natural articular cartilage, the second layer being akin to the middle layer of natural articular cartilage and the optional (but preferred) third layer being akin to the deep zone of natural articular cartilage.

The culture according to the present invention enables better environmental control to mimic physiologic conditions compared to traditional in vitro models. The three-dimensional matrix mimics the specialized ECM allowing chondrocytes to redifferentiate. Due to its similarity to natural cartilage, the chondrocyte cultures according to the present invention allow a dramatic decrease in need for animal models. In fact, each setup for modelling may easily be developed to medium- or high-throughput mode.

The following documents relate to three-dimensional cell culture or microfluidic cell culture. However, they do not anticipate or lead to the present invention.

WO 2010/101708 A2 concerns a microfluidic device and related methods for the generation of arbitrary concentration gradients within the growth medium via so-called "diffusion ports". The document is entirely silent on cartilage production. Furthermore, the document does not specifically disclose the combination of chondrocytes in a matrix which has a BSA diffusion coefficient within the range disclosed herein. The document does not even disclose the temperature at which the diffusion coefficient measurements were conducted.

US 2003/040113 A1 relates to a composition and methods for the production of biological tissues and tissue constructs. Briefly, in this method, a solution comprising suspended chondrocytes may be introduced into a sponge matrix so as to create a seeded device. The device may also contain further layers, namely an integration layer facilitating the incorporation of the device into a lesion or defect in the body and a protective layer for encapsulation. However, the document is silent on the shape of the chondrocytes and formation of cartilage layers such as the ones disclosed herein.

WO 2016/174607 A1 relates to a microfluidic device and method for the generation of three-dimensional tissue constructs. Cartilage cells may be used for this according to the disclosure. It is further disclosed that the method may also comprise compressing a cellular matrix for a predetermined period of time. However, the document is entirely silent on cartilage production and cartilage injury models.

Sticker et al. (LAB ON A CHIP 15 (24) (2015): 4542-4554) concerns multi-layered, membrane-integrated microfluidics based on replica molding of a thiol-ene epoxy thermoset for organ-on-a-chip applications. The document is entirely silent on cartilage or production thereof.

WO 2006/017176 A2 relates to scaffoldless constructs for tissue engineering of articular cartilage. According to the document, chondrocytes were introduced into hydrogel coated wells and segmented into an aggregate within 24 hours (page 5, first and second paragraph). After four weeks, the cells were still round, indicating that the chondrocytes' initial phenotype was maintained. However, layers resembling the superficial zone of natural cartilage or the deep zone of natural cartilage are not mentioned in this document. By contrast, it is essentially disclosed that flattened chondrocytes do not form at the surface (page 7, first paragraph), thereby teaching away from the present invention.

The inventive three-dimensional tissue culture is in-vitro grown, i.e. it is not an isolated cartilage from an animal. However, the chondrocytes used may be obtained from animal cartilage or a primary culture thereof. In addition, components obtained from cartilage may be used as a part of the matrix. Typically, the matrix comprises a polymer (such as an insoluble biopolymer) forming pores filled with an aqueous solution.

In the following, the three aforementioned layers of the inventive culture are described in more detail: In the first layer, the chondrocytes typically have an elongated shape that is essentially aligned in parallel to the surface and typically are in close contact to each other. In a preference, the mean sphericity of these chondrocytes is below 0.9, preferably below 0.875, more preferably below 0.85, even more preferably below 0.825 or even below 0.8, yet even more preferably below 0.775 or even below 0.75, in particular below 0.725 or even below 0.7. This indicates that the first layer is particularly structurally and functionally similar to its natural counterpart, the superficial layer. The first layer has a thickness of at least one cell, preferably at least two cells.

In the second layer, the chondrocytes typically display a round morphology and typically are essentially randomly dispersed within the layer, just as in its natural counterpart, i.e. the middle layer of natural cartilage. Typically, this layer has a larger thickness than the first layer. In a preference, the mean sphericity of the chondrocytes in this layer is above 0.9, preferably above 0.91 or even above 0.92, more preferably above 0.93 or even above 0.94, even more preferably above 0.95 or even above 0.96, especially above 0.97. Preferably, this layer has a (chondrocyte) cell density of 500-10000 cells per $mm^3$, more preferably 750-5000 cells per $mm^3$, even more preferably 1000-4000 cells per $mm^3$, especially 1250-3500 cells per $mm^3$. It is particularly preferred that the cell density of this layer is lower than the cell density of the first layer, as this further increases the similarity to natural cartilage.

In the third layer, chondrocytes are arranged into columns, as previously mentioned. The term "columns" is known in the art in connection with the deep layer of natural cartilage (i.e. a column is a linear aggregation or string of chondrocytes, typically orthogonal to the first layer) and the present invention surprisingly displays a similar behaviour. Preferably, the columns in this layer of the inventive culture have on average at least three, preferably at least four, more preferably at least five chondrocytes. Accordingly, the thickness of this layer measures at least two, preferably at least three, more preferably at least four, especially at least five chondrocyte cells. In general, the presence of this third layer increases similarity to natural cartilage, which is especially advantageous when the culture is used as an OA model or in surgery.

The three-dimensional culture of the present invention may comprise further layers, but preferably comprises only the first and the second layer or the first, second and third layer.

Herein, sphericity ($\Psi$) of a cell is defined as (see also Wadell, "Volume, shape, and roundness of quartz particles." The

$$\Psi = \frac{\pi^{\frac{1}{3}} \left(6 V_p\right)^{\frac{2}{3}}}{A_p}$$

Journal of Geology 43.3 (1935): 250-280): wherein $V_p$ is the volume of the cell and $A_p$ is the surface area of the cell. Sphericity is a dimensionless quantity. The sphericity of a perfect sphere is 1, and any cell which is not a perfect sphere will have sphericity of less than 1. Volume and surface of a cell such as a chondrocyte can for instance be measured by confocal laser fluorescence microscopy when using fluorescent cell dyes. See e.g. Amini et al. "Three-dimensional in situ zonal morphology of viable growth plate chondrocytes: A confocal microscopy study." Journal of Orthopaedic Research 29.5 (2011): 710-717, in particular Table 1, for an example of measuring the sphericity of chondrocytes.

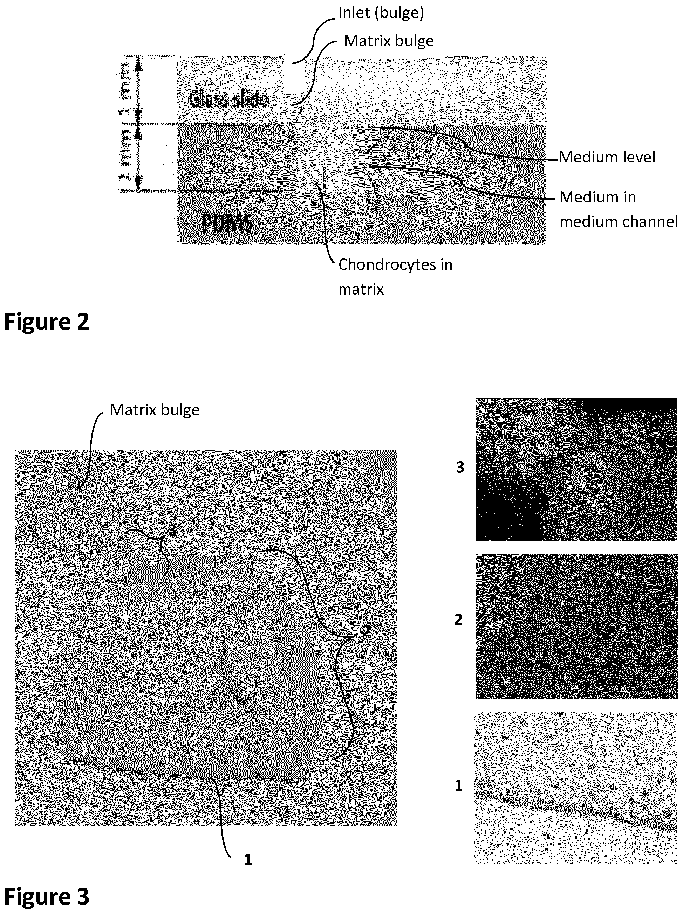

In a particularly preferred embodiment, the culture of the present invention has a Shore-A hardness score of less than 90, preferably less than 85, more preferably less than 80, even more preferably less than 75, in particular less than 70. This makes the culture especially suitable for reconstructive surgery, for instance as an articular cartilage implant. In addition, the culture preferably has a Shore-A hardness score of more than 0, preferably more than 5 or even more than 10, especially more than 10 or even more than 20, especially more than 30 or even more than 40. Shore-A hardness (i.e. Shore hardness type A) can be tested with e.g. a Shore A durometer according to the standard ASTM D2240-15 or DIN 53505:2000-08. See also Darmanis, et al. "Static indentation test for neocartilage surface hardness in repair of periosteal articular cartilage defects." Acta orthopaedica belgica 72.5 (2006): 621, especially Fig. 2.

Alternatively, or in addition thereto, the inventive culture in a preference has an indentation stiffness score of below 70, preferably between 28 and 60, as measured by a handheld ACTAEON probe, especially according to Bae, Won C., et al. "Indentation testing of human cartilage: sensitivity to articular surface degeneration." Arthritis & Rheumatism 48.12 (2003): 3382-3394, "Materials and Methods", "Indentation Testing" (p. 3384).

In a further preferable embodiment, to further improve implantability, the culture is free of at least one of the following features: tidemark, calcified cartilage and arcades of Benninghoff, optionally with subchondral bone anchorage therein; preferably free of at least two of said features, in particular free of at least three of said features. These features are usually found in naturally occurring articular cartilage and can be easily identified by the skilled artisan; see e.g. Redler, Irving, et al. "The ultrastructure and biomechanical significance of the tidemark of articular cartilage." Clinical orthopaedics and related research 112 (1975): 357-362 for tidemark; see e.g. Ferguson, et al. "Nanomechanical properties and mineral concentration in articular calcified cartilage and subchondral bone." Journal of Anatomy 203.2 (2003): 191-202 for calcified cartilage; see e.g. Wilson, W., et al. "Stresses in the local collagen network of articular cartilage: a poroviscoelastic fibril-reinforced finite element study." Journal of biomechanics 37.3 (2004): 357-366 for arcades of Benninghof. In naturally occurring articular cartilage, tidemark is conventionally defined as the distinction between the deep layer from the calcified cartilage, calcified cartilage is conventionally defined as hypertrophic chondrocytes with scarce cellularity anchoring the collagen fibrils of the deep zone to subchondral bone, and arcades of Benninghoff are conventionally defined as bundles of primary fibrils which extend perpendicular from the subchondral bone, splitting up close to the articular surface into fibrils which curve to a horizontal course, flush with the articular surface.

In a further preferred embodiment of the present invention, the matrix is at least partially composed of a biocompatible gel, preferably a hydrogel.

According to a further preference, the matrix is at least partially composed of one or more compounds selected from polyglycolic acid, hyaluronate, methylcellulose, collagen, alginate, agarose, gelatin, poly-lactic acid, fibrin, polyethylene glycol (PEG) dextran, gelatin, keratin, laminin, titin, albumin, polysaccharides, such as glycosaminoglycans, starch, cellulose, methylcellulose, dextran, hemicellulose, xylan, and chitosan, polyacrylates, polyurethane, poly-lactic-glycolic acid, polyacrylamides, PEG, PEG diacrylate (PEGDA), PEGDA-fibrinogen, polymethacrylamides, polyethyleneimines, polyvinyl resins, polylactide-glycolides, polycaprolactonces, silk fibers, carbon fibers and polyoxyethylene.

In a particular preference, the matrix is at least partially composed of a fibrin hydrogel, which is exceptionally suited to achieve a culture that is similar to natural cartilage in many aspects (see also examples).

The chondrocytes for use in the present invention are typically vertebrate chondrocytes. Preferably, they are selected from reptilian, amphibian, fish, such as zebrafish, and mammalian chondrocytes, more preferably selected from human, equine, primate, porcine, ovine, caprine, bovine and murine chondrocytes, especially human chondrocytes. It is preferred that the chondrocytes used in the present invention are obtained from a primary (preferably 2D) culture of chondrocytes from whole cartilage (i.e. not chondrocytes isolated from specific layers), preferably from a single source. In a further preference, the chondrocytes used in the present invention are directly obtained (i.e. without establishing a primary 2D culture first) from whole cartilage, preferably from a single source. The chondrocytes for use in the present invention may also be obtained by differentiating mesenchymal stem cells (MSCs), especially human MSCs (for differentiation conditions see e.g. Augello & De Bari. "The regulation of differentiation in mesenchymal stem cells." Human gene therapy 21.10 (2010): 1226-1238), preferably from a single source. Such MSCs may be obtained from the patient (such as the OA patient), e.g. in order to grow cartilage in vitro for reconstructive surgery to be performed on the patient.

In a further preferred embodiment, the longest dimension of the culture of the present invention measures 0.1 mm to 100 mm, preferably from 0.3 mm to 50 mm, more preferably from 0.5 mm to 25 mm, especially from 1 mm to 10 mm. In particular, the shortest dimension of the culture measures from 5% to 90%, preferably from 10% to 80%, more preferably from 20% to 70%, even more preferably from 30% to 60%, especially from 40% to 55%, of the longest dimension.

According to a further preferred embodiment of the present invention, the first layer forms (or is present at or close to) at least 5%, preferably at least 10%, more preferably at least 15% or even at least 20%, yet more preferably at least 30% or even at least 40%, yet even more preferably at least 50% or even at least 60%, especially at least 70% of the surface (area) of the culture. In particular, the first layer forms a single contiguous part of the surface of the culture.

It is particularly beneficial when more than 50%, preferably more than 60%, more preferably more than 70%, even more preferably more than 80%, especially more than 85% or even more than 90% of the chondrocytes of the inventive culture (i.e. of all chondrocytes present) have a sphericity of over 0.9; preferably wherein more than 50%, preferably more than 60%, more preferably more than 70%, even more preferably more than 80%, especially more than 85% or even more than 90% of the chondrocytes have a sphericity of over 0.92; more preferably wherein more than 50%, preferably more than 60%, more preferably more than 70%, even more preferably more than 80%, especially more than 85% or even more than 90% of the chondrocytes have a sphericity of over 0.94; in particular wherein more than 50%, preferably more than 60%, more preferably more than 70%, even more preferably more than 80%, especially more than 85% or even more than 90% of the chondrocytes have a sphericity of over 0.95. In a particularly preferred embodiment, the culture has reached a stable state. Accordingly, said sphericity (e.g. over 0.9) for said percentage of the chondrocytes (e.g. more than 50%) can be maintained for at least 48h, preferably for at least 72h, more preferably for at least one week, even more preferably for at least two weeks, especially for at least three weeks of culture. In this connection, it is especially preferred when only the first layer (or at least a portion thereof) is brought into direct contact with the growth medium, e.g. a chondrocyte growth medium or a chondrocyte differentiation medium.

To increase stability of the culture, it is particularly advantageous when nutrients contained in the culture (i.e. which were originally contained in the growth medium) are distributed by gradient principles, preferably with the highest concentration of nutrients in the first layer and with the lowest concentration of nutrients in the third layer.

A particular expression profile of the chondrocytes is a useful indicator that similarity to natural occurring cartilage has been achieved. Also therefore, the chondrocytes of the culture on average preferably exhibit an at least two-fold, more preferably an at least three-fold, even more preferably an at least four-fold, especially an at least five-fold, increased expression (on mRNA, as measured e.g. by RT-qPCR, or protein level, as measured e.g. by ELISA) with respect to Sox9, Coll II and/or ACAN, compared to a two-dimensional chondrocyte culture (i.e. an appropriate control, e.g. a 2D or monolayer culture of chondrocytes from the same source in the same growth medium). Furthermore, it is preferred when the chondrocytes on average exhibit an at least two-fold, more preferably an at least three-fold, even more preferably an at least four-fold, especially an at least five-fold, decreased expression (on mRNA, as measured e.g. by RT-qPCR, or protein level, as measured e.g. by ELISA) with respect to Coll I, compared to a two-dimensional chondrocyte culture (i.e. an appropriate control, e.g. a 2D or monolayer culture of chondrocytes from the same source in the same growth medium).

In a further preferred embodiment, the thickness of the first layer is 3 $\mu$m to 300 $\mu$m, wherein the thickness of the second layer is 60 $\mu$m to 3000 $\mu$m and wherein the thickness of the third layer is 60 $\mu$m to 1000 $\mu$m (e.g. to increase suitability for implantation).

It is particularly preferred when the matrix has a tensile strength between 25 kPa and 3 MPa, preferably between 30 kPa and 1 MPa. Preferably, tensile strength is measured according to ASTM F 2150-02 or when tested according to Bellucci, et al ("Mechanical behaviour of articular cartilage under tensile cyclic load." Rheumatology 40.12 (2001): 1337-1345.) in biaxial tensile cyclic loading to failure.

In a further preference, the matrix has a Young's modulus between 5 kPa and 300 kPa, preferably between 6 kPa and 200 kPa, more preferably between 7 kPa and 100 kPa, more preferably between 8 kPa and 50 kPa, even more preferably between 9 kPa and 25 kPa, yet even more preferably between 10 kPa and 20 kPa, especially between 11 kPa and 13 kPa. Preferably, the Young's modulus is measured with the unconfined compression test according to Korhonen et al (Korhonen, R. K., et al. "Comparison of the equilibrium response of articular cartilage in unconfined compression, confined compression and indentation." Journal of biomechanics 35.7 (2002): 903-909).

Furthermore, it is beneficial when the shear modulus of the matrix does not exceed 200 kPa, which is preferably measured according to Wong et al. "Biomechanics of cartilage articulation: effects of lubrication and degeneration on shear deformation." Arthritis & Rheumatology 58.7 (2008): 2065-2074, section "Microscale shear testing".

In the present invention, the matrix has a BSA diffusion coefficient of $2.5 \times 10^{-11}$ cm$^2$/s to $1 \times 10^{-6}$ cm$^2$/s, preferably $1 \times 10^{-10}$ cm$^2$/s to $7.5 \times 10^{-7}$ cm$^2$/s, more preferably $1 \times 10^{-9}$ cm$^2$/s to $5 \times 10^{-7}$ cm$^2$/s, even more preferably $5 \times 10^{-9}$ cm$^2$/s to $2.5 \times 10^{-7}$ cm$^2$/s, especially $1 \times 10^{-8}$ cm$^2$/s to $1 \times 10^{-7}$ cm$^2$/s, at a temperature of 20°C.

In a particularly preferred embodiment, the culture of the present invention is used in surgery, i.e. as implant or substitute for native cartilage (in other words, in the repair of articular cartilage or as an articular cartilage substitute). The patient undergoing surgery is preferably a vertebrate, in particular a mammal, especially a human. For instance, the patient has a joint disease such as OA.

Cells cultured in microfluidic devices are confined as compared to those cultured in monolayer, which has been shown to affect cellular differentiation augmented by shear flow (Riehl et al., Cells 1 (2012), 1225-1245). Within microfluidic devices, fluid flow is laminar, resulting in nutrient and gas exchange by diffusion and thereby allowing gradient establishment. Dedicated clean room environment and associated operating costs are virtually eliminated with microfluidic techniques. Required volume of cells, medium, and matrix are few given the scale, allowing affordability while dramatically decreasing the required experiment time. Polydimethylsiloxane (PDMS), the most commonly used microfluidic material, is gas permeable, which makes it amenable to cell culture. Devices with cells in culture can be maintained under microscopy with real time imaging. Environmental conditions are controlled with water baths, waste reservoirs and fluid pumps. Analyses can be performed on and off chip, and these modalities are often complementary to one another. Microfluidic 3D cell culture systems have been used to study cell-matrix interactions as well as paracrine signaling in co-cultures of stem cells (Hamilton et al., Biotechnol. J. 8 (2013), 485-495). Specific tissues and their relative conditions can be imposed due to the supercontrol available via microfluidics through their inherent large surface to volume ratio. Microfluidics can be used to stimulate 3D hydrogel cultures thus simulating different cellular in vivo situations including shear stress, strain and intracellular architecture (Kurth et al., Curr. Op. Chem. Biol. 16 (2012), 400-408; US 2016/0201037 A1).

It has turned out that microfluidics is particularly suitable for the purposes of the present invention, e.g. to grow or test the inventive culture. Thus, the device of the present invention is preferably a microfluidic device, such as a microfluidic chip, a live-cell micro-array or a microbioreactor. An overview about microfluidic cell culture systems in general is also given in Rosser et al., 2015 (Rosser, J. M., et al. "Recent Advances of Biologically Inspired 3D Microfluidic Hydrogel Cell Culture Systems." J Cell Biol Cell Metab 2.005 (2015): 1-14.).

The term "microfluidic" in the context of "microfluidic device" means that the device is configured to be used with small volumes of liquid, e.g. in the micro-liter-, nano-liter- or femto-liter- range. In particular, a microfluidic device of the present invention (such as a microfluidic chip) can hold a maximum volume of liquid of less than 500 µl, preferably less than 200 µl, more preferably less than 150 µl, yet more preferably less than 100 µl, even more preferably less than 50 µl, yet even more preferably less than 25 µl, especially less than 10 µl or even less than 5 µl.

Typically, the microfluidic chip of the present invention is made from a substrate (e.g. polydimethylsiloxane (PDMS) or a PDMS-glass construct or a glass-PDMS-glass sandwich), with channels etc. executed as bores, grooves (or recesses or depressions) or notches in the substrate. Typically, it is close to the shape of a flat cuboid (the flatness defining the main plane of the chip or simply "the plane of the chip") with two sides of larger area four narrow sides (of smaller area). The term "chip" refers to the characteristic typical flat appearance of the microfluidic chip. The inventive chip can also contain electronics.

The culture of the present invention on such a chip represents e.g. a microfluidic injury model capable of investigating the onset, progression and repair of biochemically and mechanically injured cartilage using a microfluidic 3D microtissue array of articular cartilage mimicking physiological environment. This chip-based cartilage injury model according to the present invention is specifically aimed to analyse the efficacy of biological and pharmaceutical treatment modalities. It may specifically be used as an osteoarthritis (OA) model. The unique combination of microfluidics with 3D cell cultures systems allows the reproducible re-engineering of the biological niche (e.g. gradients, temperature, pressure profiles), thus establishing microtissue structures that closely resemble native cartilage. Targeted biochemical or mechanical injuries using known inflammatory cytokines or compressive trauma, respectively, induce temporal and spatially resolved pathology that enables the study of the onset, progression and/or regeneration of the affected tissue. In general, the microdevice automatically establishes a biological concentration gradient that promotes the formation of functional cartilage tissue constructs and consists of a microchannel network for medium supply and compound delivery as well as a multitude of microbioreactors containing in case of the mechanical injury model a pneumatically actuated and flexible compressor.

The device according to the present invention generally automatically establishes a biological concentration gradient within 3D chondrocyte culture that promotes the formation of physiological cartilage tissue on the chip. This device is able to induce precise, reliable and reproducible biochemical or mechanical injury using microfluidics. It is therefore possible with this device to study e.g. onset, disease progression and response modulation by pharmaceutical manipulation and therapy in various cartilage pathologies, especially OA.

The device according to the present invention can be provided e.g. as a "cartilage-on-a-chip", i.e. as a 3D microfluidic device mimicking articular cartilage in vivo through tightly knit control of the cells environment including oxygenation, stress, extracellular matrix and nutrient supply. This miniaturized articular cartilage model according to the present invention allows medium-to-high throughput, real-time screening of cell morphology and viability and thus facilitates large-scale drug/ATMP (advanced therapy medicinal product) testing. In addition, cartilage-on-a-chip can help minimize the need for animal experiments as well as screening cost and time.

Preferably, the channel of the microfluidic chip of the present invention used for the growth medium has a (mean) diameter from 20-5000 µm and preferably 20-500 µm in continually perfused applications and preferably 1000-2000 µm in applications with intermittent growth medium exchange. Typically, the chip of the present invention has a chamber containing the inventive culture ("cell chamber"), which is fluidly connected to the channel for the growth medium.

According to a preferred embodiment, the inventive device (such as the microfluidic chip) comprises a channel for growth medium. In particular, a portion of the surface of the culture, preferably wherein said portion comprises or is at least a portion of the first layer, is in contact with said channel. It is especially advantageous when the channel is fluidly connectable to an outside growth medium reservoir and an outside waste reservoir (for used growth medium). In this connection, "outside" means outside of the device (especially chip).

In a further preferred embodiment, the device (in particular the microfluidic chip) is at least partially composed of a transparent solid material such as glass, PDMS or OSTEmer, preferably wherein the culture is observable from the outside through said material, preferably by a microscope.

The device of the present invention can be used as a cartilage injury model, especially as an osteoarthritis model. For instance, the culture can be studied (or "injured") using biological, chemical or physical methods including macrophage conditioned medium, osteoarthritic synovial fluid, media containing TNF-alpha and interleukin 1-beta and variations thereof, mechanical, electrical and optical injuries. The culture within the device can also be contacted with test compounds, e.g. through the channel (i.e. in the growth medium), to test their effect on cartilage injury, injury prevention or healing.

In connection with the inventive method for manufacturing, it is noted that the term "casting mould" shall not be construed as to be restricted to any particular geometric configuration, it is merely to be construed as a containment (in its broadest sense, it may e.g. also be partially open) wherein the polymerisation (or further polymerisation) of the dispersion occurs. In certain embodiments, the culture remains in the casting mould during a part or the entire culturing phase (i.e. the casting mould is a "cell chamber").

In the course of the present invention it has surprisingly turned out that it is advantageous when the casting mould has a bulge (which in turn can lead to the formation of a matrix bulge, e.g. when filled with the dispersion during polymerisation) is advantageous. Without being bound to any particular theory, especially the special hydrostatic conditions in the matrix bulge (when it is at least partially located above the level of the growth medium) are suspected to support the induction of the third layer during the culturing.

Typically, the matrix least partially extending into the bulge of the casting mould (whereby a matrix bulge is formed) is achieved by at least partially filling the bulge of the casting mould with the dispersion, e.g. during the transfer of the dispersion (e.g. by filling to a certain level such that the bulge is also filled) or after the transfer (e.g. by changing the spatial orientation of the casting mould, such as by inverting, such that the bulge is also filled). However, it can also be achieved e.g. by a dispersion that swells during polymerisation such that the matrix obtained by the polymerisation at least partially extending into the bulge of the casting mould.

Preferably, the matrix bulge has a volume of less than 40%, preferably less than 30%, more preferably less than 20%, especially less than 10% of the total volume of the matrix. If e.g. necessitated by subsequent surgical procedures (for instance if the three-dimensional tissue culture has to be fitted into a specific joint), at least a portion of the bulge (or the entire bulge or even the entire third layer which has grown, although the latter is not preferred, as it would decrease similarity to natural cartilage) can be removed (e.g. cut off) from the three-dimensional culture after the culturing.

To simplify manufacturing and handling of the casting mould, the bulge of the casting mould also serves as an inlet into the casting mould according to a preferred embodiment (i.e. the bulge can have an opening), especially as an inlet for said dispersion. Through this inlet the dispersion can be transferred into the casting mould. By way of example, the casting mould can be filled up to a level with the dispersion such that the bulge (inlet) remains at least partially filled with a portion of the dispersion during the exposing to conditions which allow polymerisation. Thereby, the matrix bulge can be formed.

According to a preferred embodiment of the present invention, the surface of the matrix bulge is at a distance to said portion of the surface of the matrix in contact with a growth medium (where nutrient concentrations are typically the highest). This further supports the formation of the third layer in the culture. Preferably, the minimal distance between the surface of the matrix bulge and said portion of the surface of the matrix in contact with a growth medium (i.e. the distance between the two surface points which are closest to each other) is at least 0.25 mm, preferably at least 0.5 mm, more preferably at least 0.75 mm, even more preferably at least 1 mm, yet even more preferably at least 1.25 mm or even at least 1.5 mm, especially at least 1.75 mm or even at least 2 mm. It is highly preferred that the surface of the matrix bulge is not in contact with a growth medium (or any liquid) during at least 50%, preferably at least 60%, more preferably at least 70%, even more preferably at least 80%, in particular at least 90% or even 100% of the culturing.

In a further preferred embodiment of the inventive method, the BSA diffusion coefficient of the matrix at 20°C is $1 \times 10^{-10}$ cm$^2$/s to $7.5 \times 10^{-7}$ cm$^2$/s, preferably $1 \times 10^{-9}$ cm$^2$/s to $5 \times 10^{-7}$ cm$^2$/s, more preferably $5 \times 10^{-9}$ cm$^2$/s to $2.5 \times 10^{-7}$ cm$^2$/s, especially $1 \times 10^{-8}$ cm$^2$/s to $1 \times 10^{-7}$ cm$^2$/s. This further supports the layer formation in the culture.

Preferably, the chondrocytes are obtained from a primary culture of cartilage (in particular whole cartilage), preferably cartilage isolated from a vertebrate, more preferably isolated from a reptile, amphibian, fish, such as zebrafish, or a mammal, even more preferably from a human, horse, primate, pig, sheep, goat, cow or mouse, especially from a human. Said vertebrate (especially human) may also be a deceased individual.

In a particular preference, at least 50%, preferably at least 60%, more preferably at least 70%, even more preferably at least 80%, yet even more preferably at least 90%, especially at least 95% or even 99% or even all chondrocytes provided for the method (or present in the culture of the present invention) are obtained from the same primary culture.

Typically, this primary culture is a 2D (or monolayer) culture. Alternatively, the primary culture may be a 3D culture.

In an alternative preferred embodiment, the chondrocytes are directly obtained from cartilage (preferably whole cartilage) isolated from a vertebrate, more preferably isolated from a reptile, amphibian, fish, such as zebrafish, or a mammal, even more preferably from a human, horse, primate, pig, sheep, goat, cow or mouse, especially from a human. Said vertebrate (especially human) may also be a deceased individual. In particular, at least 50%, preferably at least 60%, more preferably at least 70%, even more preferably at least 80%, yet even more preferably at least 90%, especially at least 95% or even 99% or even all chondrocytes provided for the method (or present in the culture of the present invention) are obtained from the same individual.

In a preferred embodiment, the polymerisable molecules comprise a protein or a polysaccharide, preferably the polymerisable molecules comprise fibrinogen.

A suitable matrix can also be achieved in particular with one or more of the following polymerisable molecules: polyglycolic acid, hyaluronate, methylcellulose, collagen, alginate, agarose, gelatin, poly-lactic acid, fibrin, fibrinogen, PEG dextran, gelatin, keratin, laminin, titin, albumin, polysaccharides, such as glycosaminoglycans, starch, cellulose, methylcellulose, dextran, hemicellulose, xylan, and chitosan, polyacrylates, polyurethane, poly-lactic-glycolic acid, polyacrylamides, PEG, PEGDA, PEGDA-fibrinogen, polymethacrylamides, polyethyleneimines, polyvinyl resins, polylactideglycolides, polycaprolactonces, silk fibers, carbon fibers and polyoxyethylene.

Herein, the term "polymerisation" also comprises polymerisation by a change in temperature (such as achieved by basement membrane-like matrix, e.g. MATRIGEL®) and/or crosslinking of polymers, i.e. also a polymer may be a polymerisable molecule. In some embodiments, exposure to conditions that allow polymerisation (e.g. conditions comprising addition of thrombin when the polymerisable molecule is fibrinogen) may already be started before the dispersion has been transferred to the casting mold. Of course, in this case, polymerisation should be sufficiently slow (and still ongoing), such that the dispersion is still easily transferable into the casting mould (and is for instance not too viscous for this).

In a particularly preferred embodiment, the polymerisation comprises an enzymatic polymerisation (which typically is less detrimental to the chondrocytes present in the dispersion), such as a polymerisation by thrombin. If the polymerisable molecule is fibrinogen, thrombin is preferably added up to a concentration of at least 0.1 IU/ml, preferably at least 1 IU/ml, more preferably at least 2 IU/ml, especially at least 3 IU/ml or even at least 5 IU/ml.

A particularly suitable matrix is obtained when the dispersion contains 0.5-70 mg/ml or even 1-65 mg/ml, preferably 2.5-60 mg/ml or even 5-55 mg/ml, more preferably 10-50 mg/ml, even more preferably 12.5-40 mg/ml, yet even more preferably 15-30 mg/ml, especially 15-25 mg/ml or even 17-19 mg/ml fibrinogen; preferably mammalian fibrinogen, especially human fibrinogen (e.g. TISSEEL® by Baxter International, Inc., Deerfield, USA).

For the same reason, it is beneficial when the dispersion contains chondrocytes at a concentration of 100-15000 cells per $mm^3$, preferably 500-10000 cells per $mm^3$, more preferably 750-5000 cells per $mm^3$, even more preferably 1000-4000 cells per $mm^3$, especially 1250-3500 cells per $mm^3$.

In a further preference, the growth medium is a growth medium comprising a transforming growth factor (TGF)-beta (e.g. TGF-beta3), preferably a chondrocyte differentiation medium. Chondrocyte differentiation medium is for instance available from Lonza Group Ltd, Basel, Switzerland (Catalog # PT-3925, PT-4121 and PT-4124) or from Thermo Fisher Scientific Inc., Waltham, USA (StemPro® Chondogenesis Differentiation Kit, Gibco®, catalog no. A10071-01).

According to a further preferred embodiment, said portion of the surface of the matrix is 1%-99%, preferably 2%-95% or even 3%-90%, more preferably 5%-85% or even 7.5%-80%, even more preferably 10%-70% or even 15%-60%, yet even more preferably 20%-50%, especially 30%-40% of the total surface of the matrix.

The casting mould is a cell chamber of a microfluidic chip and said growth medium is brought in contact with said portion of the surface of the matrix through a medium channel of the microfluidic chip during the culturing; preferably wherein said channel is fluidly connected to an outside growth medium reservoir and an outside waste reservoir.

According to a further preferred embodiment, the culturing is performed for at least 48h, preferably for at least 72h, more preferably for at least one week, even more preferably for at least two weeks, especially for at least three weeks of culture; preferably wherein said culturing is performed in a microfluidic device such as a chip and/or wherein the growth medium is exchanged periodically or continuously (with fresh growth medium), such as by perfusion through a medium channel of a microfluidic chip. In particular, the culturing is performed until the culture of the present invention (i.e. the culture with the three layers, as defined herein) is obtained.

In a preference, for perfusion, the average fluid flow rate is 0.1 - 10 µl per hour, preferably 0.25 - 5 µl per hour, especially 0.45 - 2 µl per hour.

A further preference of the present invention stipulates that the chondrocytes in the matrix are subjected to mechanical stimulation, preferably while in the cell chamber of the microfluidic chip.

Herein, the term "BSA diffusion coefficient" in respect to a matrix (e.g. "wherein the matrix has a BSA diffusion coefficient of X") means the diffusion coefficient of BSA in the matrix when the matrix is soaked with an aqueous solution (e.g. by being submerged in a physiological buffer such as phosphate buffered saline (PBS) or in a growth medium such as chondrocyte growth medium or by being in contact with a physiological buffer such as PBS or in a growth medium such as chondrocyte growth medium present in a medium channel of a microfluidic chip). This diffusion coefficient may be the diffusion coefficient at "infinite dilution" of BSA e.g. by means of extrapolation. However, in view of practical considerations (see also next paragraph), it is preferably the diffusion coefficient at a low concentration of BSA such as 25-125 µg/ml BSA (e.g. 125µg/ml BSA) .

Preferably, the BSA diffusion coefficient values as used herein shall be accorded an error margin of ±25% or ±10% or ±5% (preferably ±25%). The BSA used for the diffusion assay may be fluorescein isothiocyanate (FITC)-conjugated BSA (which is commercially available). While the conjugation with FITC (MW=389 Da) leads to an increase of molecular mass of BSA (66 kDa), such increase is typically negligible in view of the error margin applied by the skilled person to a diffusion coefficient measurement. The BSA diffusion coefficient may be measured as disclosed in Shkilnyy et al. (Shkilnyy, Andriy, et al. "Diffusion of rhodamine B and bovine serum albumin in fibrin gels seeded with primary endothelial cells." Colloids and Surfaces B: Biointerfaces 93 (2012): 202-207)), in particular item 2.4 (with the exception of the temperature being set to 20°C instead of 22°C, although the influence of this temperature difference is typically negligible in view of the above error margin). Of course, any other suitable method known in the art can be used.

The term "growth medium" as used herein shall be defined as any liquid culture medium sufficient for growth (i.e. an increase in number) of chondrocytes, preferably vertebrate chondrocytes, in particular mammalian chondrocytes such as human chondrocytes. Suitable media are well-known in the art, e.g. from Gosset et al. (Gosset, Marjolaine, et al. "Primary culture and phenotyping of murine chondrocytes." Nature protocols 3.8 (2008): 1253.) or from Kamil et al. (Kamil, S. H., et al. "Tissue engineered cartilage: utilization of autologous serum and serum-free media for chondrocyte culture." International journal of pediatric otorhinolaryngology 71.1 (2007): 71-75.). (Preferably, this growth medium is a chondrocyte growth medium (such as provided by PromoCell GmbH, Heidelberg, DE, catalog number C-27101), or chondrocyte differentiation medium.

Herein, the term "growth conditions" refer to any conditions which allow growth of chondrocytes. Such conditions are well known in the art (see e.g. Gosset et al., full citation above). For instance, for human chondrocytes, optimal growth conditions comprise incubating the chondrocytes in chondrocyte growth medium at a temperature of 37°C and in an atmosphere with 5% (v/v) $CO_2$.

The present invention is further described by the following examples and the figures, yet without being restricted thereto.

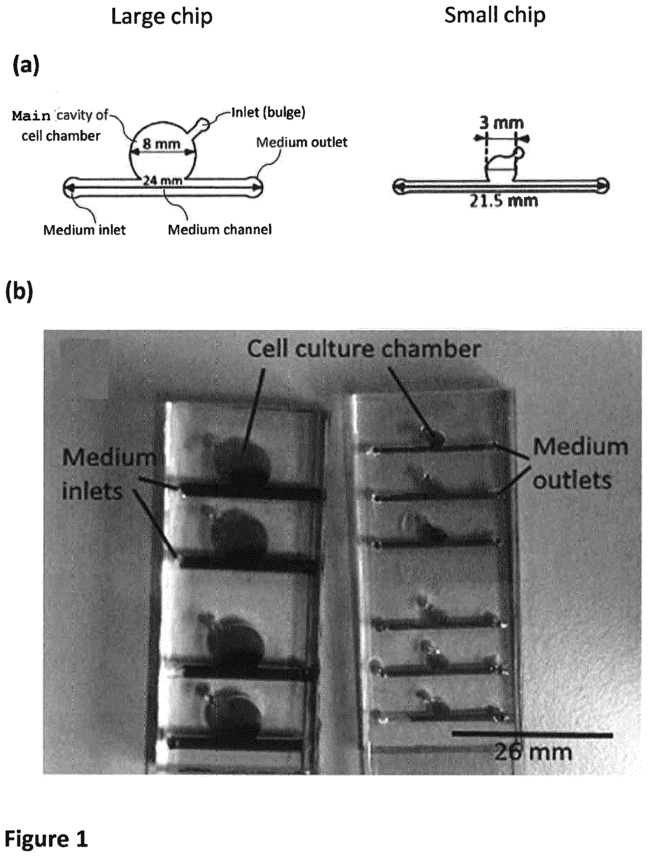

Fig. 1: Small and large microfluidic chips. (a) Schematic top view and (b) photograph of the small and large chips used for culturing chondrocytes in an artificial fibrin hydrogel matrix. The cell chamber also serves as casting mould to be filled with the dispersion comprising chondrocytes, the bulge of the casting mould has an opening and thereby forms the inlet. As evident from the photograph, a chip can comprise several cell chambers.

Fig. 2: Schematic side section of the microfluidic chip. Shown is a schematic side section of a microfluidic chip comprising the chondrocytes in the matrix in the cell chamber (casting mould) under growth conditions. The matrix extends at least partially into the bulge of the casting mould. The medium channel is filled with growth medium, and the matrix bulge is located above the level of the growth medium ("medium level").

Fig. 3: The inventive culture containing three layers. Depicted is a micrograph of a histological cross-section of the inventive culture (which was grown in a microfluidic chip according to the method of the present invention) (large image, small image "1") and a fluorescence micrograph of the inventive culture grown under the same conditions (small images "2", "3"). Chondrocytes were stained and appear as coloured spots. From the images, it becomes evident that the culture comprises three layers: (1) first layer (akin to the superficial zone of natural cartilage), which established itself at the surface of the matrix that was in contact with growth medium. The chondrocytes have an elongated morphology oriented along the surface; (2) second layer (akin to the middle zone of natural cartilage), which is located between the first and the third layer, has a lower cell density than the first layer (about 1500 cells/mm$^3$), and in which the chondrocytes display a round morphology (i.e. their sphericity is higher than of the chondrocytes in the first layer); and (3) third layer (akin to the deep zone of natural cartilage), where the chondrocytes form columns extending into the matrix (the main axis of these columns is more or less perpendicular to the plane of the surface of the first layer).

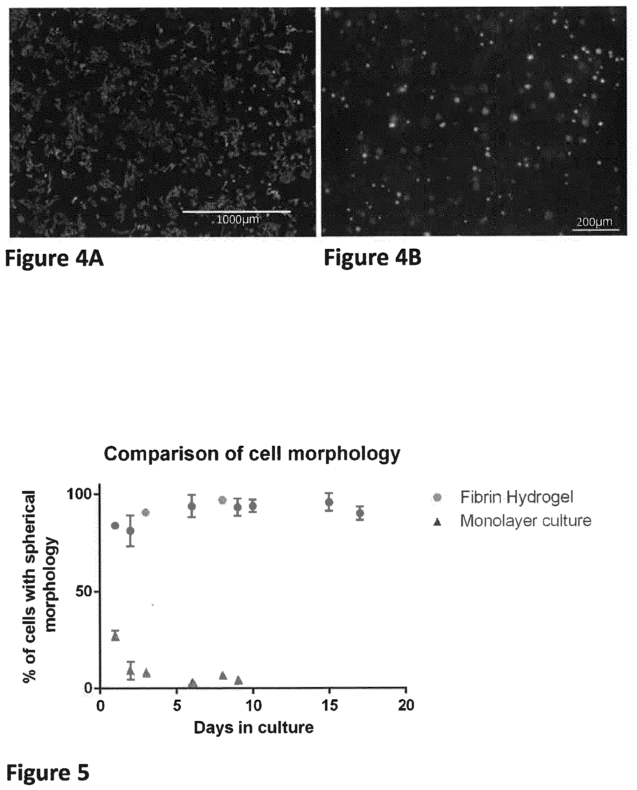

Fig. 4: Cell morphology in 2D vs. 3D culture of chondrocytes. Depicted are fluorescence micrographs with stained chondrocytes. (A) When chondrocytes were grown in monolayer, an elongated, fibroblast-like shape was observed. (B) By contrast, chondrocytes grown in a three-dimensional artificial matrix were able to re-differentiate to a characteristic round shape.

Fig. 5: Cell morphology in 2D vs. 3D culture of chondrocytes over the culture period. As the number of days in culture increases, the differences between 2D and 3D culture as shown in Fig. 4 become more pronounced.

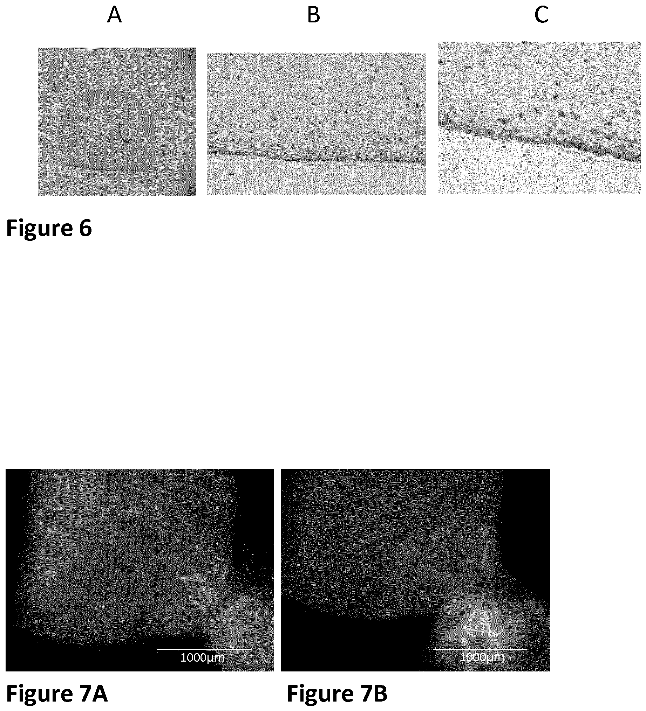

Fig. 6: Cross-section of the inventive culture. Shown are micrographs of a histological cross-section of the inventive culture (which was grown in a microfluidic chip according to the method of the present invention) with different magnifications (A-C). Chondrocytes appear coloured due to staining.

The first and the second layer are clearly visible.

**Fig. 7: Fluorescence micrographs of the inventive culture.** Fluorescence micrographs of the inventive culture (which was grown in a microfluidic chip according to the method of the present invention) are shown. Chondrocytes appear coloured due to fluorescent staining. With chondrocyte seeding densities of both (A) 3000 cells/mm3 and (B) 1500 cells/mm3, the formation of the third layer (with columns of chondrocytes clearly discernible) adjacent to and in the matrix bulge could be achieved.

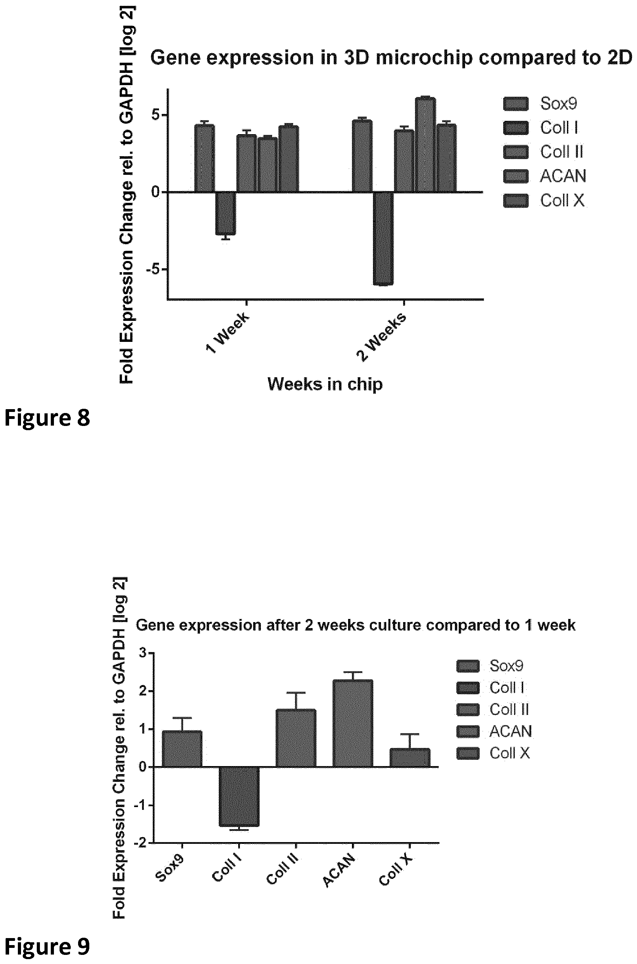

**Fig. 8: Gene expression in 2D vs. 3D culture of chondrocytes.** Shown is the difference in expression level between chondrocytes grown in 3D culture vs. chondrocytes grown in 2D culture. The expression of genes characteristic for differentiated chondrocytes is strongly increased and becomes more pronounced with increasing culture period.

**Fig. 9: Gene expression in 3D culture of chondrocytes.** Like Fig. 8, this figure shows that the effect (expression of characteristic chondrocyte genes) becomes more pronounced with time in 3D culture.

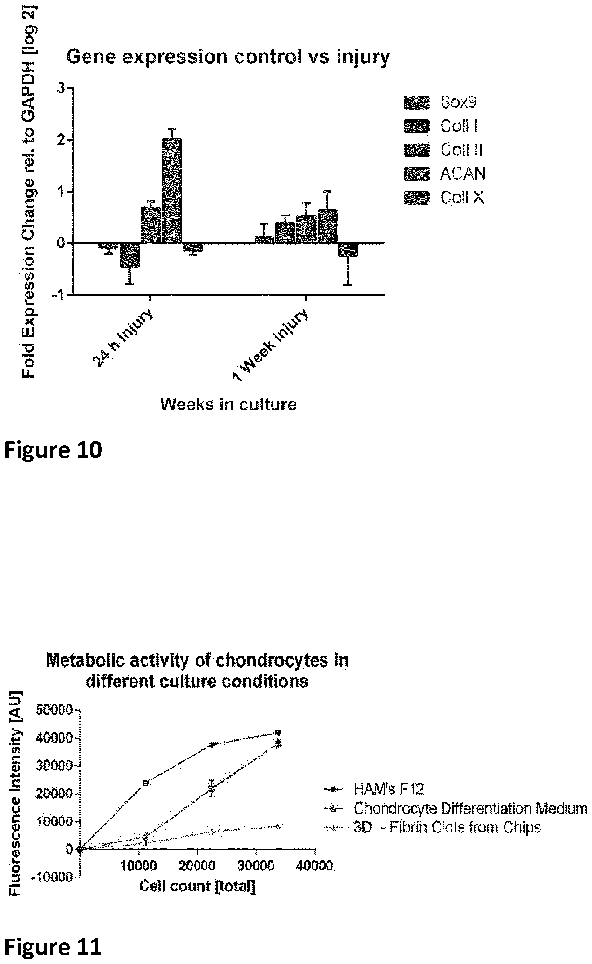

**Fig. 10: Gene expression in 3D culture of chondrocytes after chemical injury.** Results show an increase in SOX9 and collagen I as well as a decrease in aggrecan and collagen II at the 1-week timepoint compared to 24hr post injury. Collagen X was downregulated vs. control in both injury timepoints.

**Fig. 11: Metabolic activity of chondrocytes in different culture conditions.** The metabolic activity of chondrocytes cultured in 3D culture on a chip was significantly lower than the one of chondrocytes cultivated on monolayer. This shows that chondrocytes on chip resemble the in vivo situation of cartilage more accurately, in view of the low proliferative and metabolic activity also observed in in vivo chondrocytes.

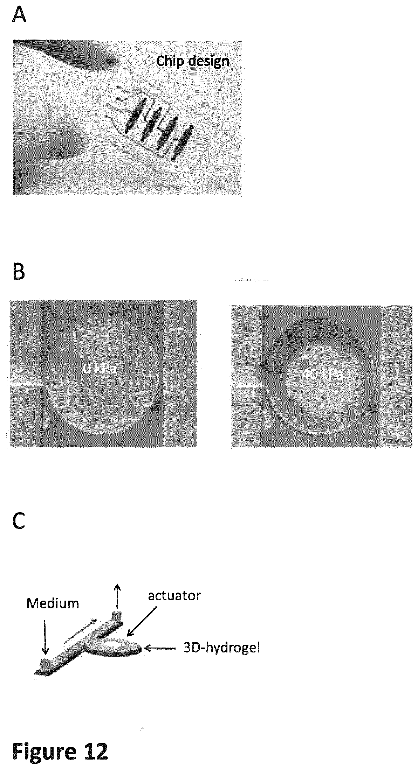

**Fig. 12: Mechanical stimulation of the chondrocyte culture in a microfluidic chip.** Schematic layout of a microfluidic cartilage-microarray containing an integrated pneumatically actuated flexible membrane to provide compressive stress situations within the circularly demarcated area.

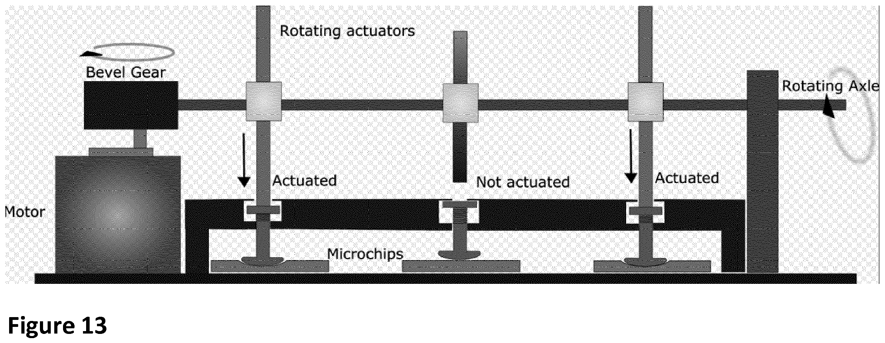

**Fig. 13: Mechanical stimulation of the chondrocyte culture in a microfluidic chip** - **alternative embodiment.** Schematic of device for providing external compressive stimulus to the PDMS surface of a microfluidic device comprising the chondrocyte culture.

**Example 1** - Microfluidic chip containing a casting mould which also serves as a cell chamber:

The microfluidic chip consists of a glass top layer with inlets and a PDMS bottom layer which can be opened to release the three-dimensional tissue culture e.g. for histological analysis. To investigate whether chamber size made a difference on the behaviour of the chondrocytes and the imaging properties of the 3D matrix, two devices were designed as shown in Fig. 1, with dimensions of: 3 mm cell chamber diameter, 7.5 microliter chamber volume, growth medium channel of 21.5 mm and 1 mm in height; and chamber diameter of 8 mm, chamber volume of 110 microliters, growth medium channel of 24 mm and 2 mm in height, respectively.

The chips were manufactured as follows: Moulds for soft lithography of PDMS were designed using AutoCAD software and manufactured by stereolithography (imaterialise). The soft lithography mould was cleaned using 99 percent isopropanol and dried at 70 degrees Celsius. Polydimethylsiloxane (PDMS, Sylgard® 184 Silicone Elastomer Kit, Down Corning) polymer was then mixed in a 1:10 ratio of curing agent and base, distributed evenly on the surface of the mould and polymerized at 70 degrees Celsius for one hour. Inlets on the glass cover slides were drilled using a 1 mm spheroid diamond drill bit to form the top layer. Prior to plasma activation, both layers were again cleaned with isopropanol and dried at 70 degrees Celsius. After drying, substrates were plasma activated for 45 seconds each using a handheld corona plasma discharge system to create excess hydroxyl groups on both surfaces and ensure stable adhesive bonding. The two layers were then aligned with one another and gentle pressure applied prior to overnight incubation.

In subsequent experiments, both chip designs ("large chip" and "small chip", cf. Fig. 1) turned out to be well-suited for growing the inventive three-dimensional culture with the three layers.

**Example 2** - Isolation and culture of primary equine chondrocytes as chondrocyte source for the inventive method:

Primary chondrocytes were isolated with written owner consent from equine patients euthanized for reasons unrelated to osteoarthritis. After shaving the area free from hair and under strict sterile technique, lateral parapatellar arthrotomy was performed with the limb in flexed position. The articular cartilage from the medial and lateral femoral trochlear ridges, the intertrochlear groove and the patella was removed in full thickness fashion using a number 10 Bard Parker scalpel and stored in sterile physiologic buffered saline (PBS). After harvest, the cartilage was cut into small pieces and digested in collagenase from Clostridium histolyticum (Sigma Aldrich) for 6 hours at 37 degrees Celsius while stirring. The digest was filtered using a cell strainer (100 micrometer, Greiner BioOne) and washed twice with PBS (Lonza) between centrifugation steps at 440 rpm for 5 minutes. Cells were then suspended in HAM's F12 complete chondrocyte medium, and cultured in polystyrene tissue culture flasks (Sarstedt). Isolated chondrocytes were grown to 80 percent confluency, detached from the culture flask using trypsin-EDTA solution (0,05 percent Trypsin/0,02 percent EDTA, Biochrom) prior to cryopreservation using freezing medium and a freezing rate of -1 degree Celsius/minute using a Nalgene® cryo freezing container and stored in liquid nitrogen until further use.

**Example 3** - Manufacturing the inventive three-dimensional tissue culture comprising chondrocytes in a microfluidic chip

Providing chondrocytes: Chondrocytes obtained according to example 2 were thawed, washed twice with PBS and subsequently cultivated in monolayer in tissue culture polystyrene flasks until 80 percent confluency. After passage two or three, the chondrocytes were provided for the dispersing step.

Dispersing the chondrocytes: Immediately prior to transfer into the casting mould (serving as a cell chamber) of a microfluidic chip obtained according to example 1, the chondrocytes were washed twice with PBS and stained for 45 min using 1 $\mu$M cytoplasmic Cell Tracker Green™ CMFDA Dye (Thermo Fisher) in pure HAM's F12 medium to facilitate cell imaging inside the turbid fibrin hydrogel matrix through several generations. After staining, the chondrocytes were washed twice with PBS, detached using 0.25 percent trypsin-EDTA solution and centrifuged at 450 rpm for 5 minutes. The supernatant was discarded and the cell pellet was resuspended in a minimal amount of chondrocyte medium to yield a concentrated cell suspension. Cell concentration and viability were determined using via Trypan Blue exclusion using Countess™ automated cell counter (Invitrogen). A homogenous dispersion was obtained by mixing the concentrated cell suspension with a fibrinogen solution and a thrombin solution containing calcium chloride (both proteins commercially available from Baxter International, Inc., e.g. in the form of TISSEEL® fibrin sealant). In the homogenous dispersion, the final fibrinogen concentration was 18 mg/ml, the final thrombin concentration was 25 IU/ml and the final chondrocyte concentration was 1500 cells/mm$^3$ or, alternatively, 3000 cells/mm$^3$.

Transferring the dispersion into the casting mould (cell chamber) of the chip: Immediately, the homogenous dispersion was transferred into casting mould (cell chamber) of the chip. The inlet formed the bulge of the casting mould. The dispersion was filled to a level such that not only the main cavity of the casting mould but also almost all of the inlet was filled with the dispersion. For the large chip, the volume of the dispersion used for filling was 110 $\mu$l, for the small chip, it was 7.5 $\mu$l.

Exposing the dispersion in the casting mould to conditions which allow polymerisation: The dispersion was allowed to polymerize through the enzymatic action of thrombin on fibrinogen at 37 degrees Celsius in a cell culture incubator, thereby obtaining a matrix comprising a fibrin hydrogel, with chondrocytes dispersed therein, with a matrix bulge present in the inlet (see Fig. 2). The BSA diffusion coefficient of this matrix at 20°C is 1 × 10$^{-7}$ cm$^2$/s. (Diffusion measurements with FITC-BSA were performed with a fibrin hydrogel matrix produced in the same way.)

Culturing: After the polymerization, chondrocyte differentiation medium (StemPro® Chondogenesis Differentiation Kit, Gibco®, catalog no. A10071-01, Thermo Fisher Scientific Inc., Waltham, USA) was added into the medium channel of the chip. Microfluidic inlets of the chip (including the inlet comprising the matrix bulge) were sealed with clear, self-adhesive foil (Polyolefin-StarSeal Xtra Clear - StarLab). The chip was incubated for 21 days at 37°C and 5% (v/v) CO$_2$. The medium of the medium channel was exchanged by manual pipetting every other day.

The culture obtained as described above has a Shore-A hardness score of less than 70 and is free of the following features: tidemark, calcified cartilage and arcades of Benninghoff.

With the method described in this example, the inventive culture comprising the three layers was obtained (see Figs. 3, 6, and 7). Dispersions with both cell densities (1500 cells/mm$^3$ or, alternatively, 3000 cells/mm$^3$) were each tested in large and small chips. The inventive culture was obtained with all of these variations.

**Example 4** - Comparison of 2D and 3D cultures

As a control, two-dimensional (2D) chondrocyte cultures were seeded in 6-well plates using a seeding density of 5000 cells/cm$^2$, to enable similar cultivation times as in the microfluidic chips, and cultivated in HAM's F12 complete chondrocyte medium as well as separately in chondrocyte differentiation medium, both with respective medium changes twice/week. Compared to the control, the portion of chondrocytes cultured on a chip showing a typical spherical morphology was significantly higher from day one and increased up to 99 percent of re-differentiated cells with prolonged time of culture while the chondrocytes cultured on a flat tissue surface became more de-differentiated during the culture period, shown by a decrease in round cells down to 3 percent. See Figs. 4 and 5.

Furthermore, histological differences between three-dimensional cultivation in fibrin hydrogels on a chip and a cultivation off chip (where the entire surface of the three-dimensional matrix with the chondrocytes is in contact with growth medium) were investigated by seeding cells onto fibrin hydrogels of 65 microliter volume into a 96-well tissue culture plate to achieve 2 mm hydrogel height and cultivation of the hydrogel matrices for 3 weeks using chondrocyte differentiation medium. It became clear that the cell viability of cells cultivated off-chip markedly decreased during the culture period as the amount of fluorescent cells and overall fluorescence declined throughout the culture period. Histological assessment of the off-chip matrices revealed a de-differentiated, dense cell layer in the upper part of the gel and a decreasing cell density towards the bottom. This decrease in differentiated chondrocytes and overall cell density could be due to limited nutrient supply and migration of the cells towards the medium.

In addition, the chondrocytes in the large and small chips were closely monitored throughout a culture period of up to 4 weeks in order to assess potentially decreased cell viability or unanticipated incompatibility with the microfluidic materials. The viability of the cells was determined by means of Cell Tracker Green™ CMFDA Dye, which is a green fluorescent dye only retained in the cytoplasma of living cells. The chondrocytes showed constant high fluorescence and thus cell viability during the entire culture period. Furthermore, size of the cell chamber did not influence cell viability.

**Example 5** - Live cell imaging

Chondrocytes cultivated in fibrin hydrogel as well as in conventional cell culture were imaged via brightfield, phase contrast and fluorescent microscopy throughout the entire culture period of 21 days using an EVOS cell imaging system (Thermo Fisher). Morphology as well as viability were assessed by means of Cell Tracker Green™ CMFDA Dye and the cells were counted every day in multiple microfluidic chambers and culture vessels cultured in HAM's F12 chondrocyte medium to compare the morphological re-differentiation process of the chondrocytes during the culture period. Chondrocytes were considered re-differentiated when they displayed a round, spherical morphology and fibroblastic when their length was twice their width. If the morphology differed from those specifications, the differentiation status was listed as not assigned.

**Example 6** - Histology

Histological cross-sections of chondrocyte-laden fibrin hydrogels cultivated either inside the microfluidic device or in a 96-well plate were performed to determine differences in cell-hydrogel structure as well as cell morphology and distribution. After cultivation, the hydrogels were fixed overnight using 4 percent formalin, released from the microfluidics using a scalpel and tweezers and kept in histology cassettes in 70 percent ethanol until embedding. The hydrogels were embedded in paraffin using a Shandon Tissue Excelsior (Thermo Fisher Scientific, Waltham, MA, USA) after an ascending alcohol series and dehydration with xylene and cut in slices of 2 microliter thickness. Next, the cuts were deparaffinated and rehydrated using a descending alcohol series before staining with Haematoxylin (Richard Allan Scientific, Waltham, MA, USA) for six minutes and Eosin (Carl Roth, Karlsruhe, Germany) for five minutes. After staining, the cuts were again dehydrated using an ascending alcohol series and mounted using DPX (Sigma Aldrich, St. Louis, MO, USA).

**Example 7** - Metabolic activity

A resazurin based in vitro toxicology assay (TOX8) was performed to determine differences in metabolic activity between the different culture formats. In order to assess the importance of different cell counts, triplicates of 11,250 cells, 22,500 cells and 33,750 cells per well were seeded in a 24-well plate for 2D control, in addition to TOX8 quantification for the 3D microfluidic culture at a cell density of 1500cells/mm$^3$. The cells were cultured for six days either with the standard cultivation method using HAM's F12 complete chondrocyte medium or with chondrocyte differentiation medium to get a better idea of the metabolic changes during differentiation. To cancel out variations caused by different liquid volumes of the microfluidic compared to 2D cultivation, the fibrin clots were released from the microfluidic at day 6 of culture and placed in a 24-well plate with either one, two or three fibrin clots per well. For the assay, 40 microliters of TOX8 reagent (Sigma Aldrich) was added to 400 microliter medium and incubated for 8 hours at 37 degrees Celsius inside a cell culture incubator. The readout was performed by aliquoting 100 microliter of supernatant per technical triplicate in a flat-bottom 96-well plate and measured fluorometrically at a wavelength of 590 nm with excitation at 560 nm.

The metabolic activity of chondrocytes cultured on a chip was significantly lower than the one of chondrocytes cultivated on monolayer. This can either be due to a higher status of differentiation of the chondrocytes embedded within the hydrogel or to higher cell numbers in of chondrocytes in monolayer due to proliferation during the six-day culture period previous to the experiment. Both explanations support the fact that chondrocytes on chip resemble the in vivo situation of cartilage more accurately, in view of the low proliferative and metabolic activity also observed in in vivo chondrocytes. See also Fig. 11.

**Example 8** - Gene expression analysis

To determine the re-differentiation capability of chondrocytes cultured in a three-dimensional fibrin hydrogel on a chip and to compare the expression profiles of chondrocytes cultured off chip in 3D to chondrocytes cultured in conventional 2D culture, total RNA was extracted from cell-laden fibrin hydrogels and 2D controls cultured in HAM's complete chondrocyte medium as well as chondrogenic differentiation medium on days 1, 7 and 13 of culture. Fibrin hydrogels with a volume of 110 microliters were released from the chip and digested in 100 IU/mL Nattokinase (JBSL-USA) for 45 min at 37 degrees Celsius with repeated mixing. The digest was centrifuged at 550 rpm for 5 minutes, the supernatant discarded and the resulting pellet used for RNA extraction. Total RNA was extracted from both 3D and 2D cultures using Peqgold total RNA isolation kit (Peqlab) per the provided protocol. The purified total RNA samples were stored at -80 degrees Celsius until further use.

Prior to cDNA synthesis, the samples were thawed on ice and the amount and purity of RNA were quantified using NanoDrop. An EasyScript™ cDNA Synthesis Kit (Applied Biological Materials Inc.) was used to synthesize the cDNA. The pre-RT reaction mix was prepared following the suppliers' instructions and incubated at 65 degrees Celsius for 5 min in a thermal cycler (Eppendorf Mastercycler) before addition of ribonuclease inhibitor and reverse transcriptase to make up the final RT mix. The resulting mix was centrifuged briefly to combine all of the ingredients at the bottom of the tube and then incubated for 10 min at room temperature. After the incubation, cDNA was synthesized through incubation of the reaction mix at 42 degrees Celsius for 40 min and subsequent cooling at 4 degrees Celsius for at least 5 minutes. The cDNA samples were stored at -20 degrees Celsius until preparation of RT-qPCR master mix.

KAPA SYBR FAST Kit was used to perform RT-qPCR (Peqlab) to quantify the gene expression profiles of markers specific for chondrogenic differentiation. Prior to analysis, standardization of the designed primers was performed using purified total RNA of primary chondrocytes directly after collagenase digestion in order to assess the primer concentration needed for a successful RT-qPCR reaction as well as the quality of the primers.

The reaction mix including the synthesized cDNA, the designed primers and the Kapa SYBR Fast Master Mix was prepared according to the manufacturer's instructions. Each RT-qPCR was run in technical triplicates for each sample and target gene using a Stratagene Mx3005P thermal cycler. The program used for quantification was a normal 2-step program with 5 minutes at 95 degrees Celsius to initiate the reaction followed by 40 subsequent cycles of 30 seconds at 95 degrees Celsius and 1 minute at 60 degree Celsius and a final determination of the melting curve with 1 minute at 95 degrees Celsius, 30 seconds at 55 degrees Celsius and 30 seconds at 95 degrees Celsius. The Ct value was used to determine the fold expression change of the target. Samples were either calibrated using the Ct value at day one of cultivation on chip or the Ct value of chondrocytes cultures in 2D monolayer for the same culture period.

Genes for expression of cartilage matrix proteins aggrecan (ACAN) and collagen II (Coll II) as well as a marker for chondrogenic differentiation (Sox9) were tested on both culture formats. The dedifferentiation status of the cells was assessed by measuring the fold change of expression of the gene Coll I, since an increase of collagen I expression and a decrease of collagen II expression is the main marker for dedifferentiation of chondrocytes. For the analysis, the fold expression changes in 3D culture were referenced with the fold expression changes in monolayer culture. The results thus represent the expression change in 3D culture compared to 2D culture with the 2D values representing zero. The graph shows the log2 of the fold expression change, for example representing an almost 60-fold higher expression of ACAN and a decrease in expression of collagen I down to 62-fold. All chondrogenesis markers were significantly increased in 3D fibrin hydrogel culture and collagen I was significantly decreased in chondrocytes on chip, both supporting the earlier observations of successful redifferentiation of chondrocytes on chip.

Additional to evaluation of differences in expression patterns between cells cultured in monolayer and in 3D hydrogel culture, the changes in expression pattern can also be used to assess the changes in gene expression with prolonged culture times. This helps to determine whether longer culture periods are necessary to develop a functional microtissue for in vitro testing. The expression of the cells after a culture period of 2 weeks was referenced to the expression after 1 week of culture, the graph thus presents the change in gene expression between 1 week and 2 weeks of culture.

A prolonged culture time results in increase of all expression of all genes related to cartilage formation and an even further decrease in expression of collagen I. This not only supports the finding that the chondrocytes have a high potential of re-differentiation when brought back to a three-dimensional culture interface but also shows that an increase of culture time on chip assists the establishment of functional cartilage microtissues. See also Figs. 8 and 9.

**Example 9** - Cartilage injury model

A 3D chondrocyte culture was injured biochemically by adding medium with 50 pg/mL TNFα and IL - 1β (see Sun et al., Biomaterials 32 (2011), 5581-5589 for details on a cartilage injury model). Instead of continuous injury, the cells were subjected to repeated periods of injury, followed by periods of cultivation without inflammatory cytokines to mimic the repeated injury process leading to an osteoarthritic phenotype in vivo for determination of a regenerative process. The cells were first subjected to biochemical injury for 24 hours after six days in culture. After this first injury period, cells were allowed to regenerate for 24 hours. In summary, the cells were injured at day 6, 8 and 11 of culture with periods of regeneration day 7 and 9 and final collection of supernatant at day 13. The supernatant of every timepoint was evaluated using off-chip time-resolved biomarker analysis and gene expression was analyzed at day 1, 7 and 13. See Fig. 10 for results.

**Example 10** - Manufacturing the inventive three-dimensional tissue culture comprising chondrocytes in a microfluidic chip under different conditions

The inventive three-dimensional tissue culture with the first, second and third layer was successfully manufactured when performing the steps disclosed in example 3, even when certain conditions had been changed:
The inventive three-dimensional tissue culture was successfully obtained in chips with a geometry identical to the chip of example 3 (i.e. with a bulge) but with different cell chamber heights. Specifically, cell chambers with heights of 250 $\mu$m, 500 $\mu$m, 1000 $\mu$m and 2000 $\mu$m all led to the desired result.

The same was true for different chondrocyte densities in the dispersion, specifically chondrocyte densities of 1,500 cells/mm$^3$, 3000 cells/mm$^3$, and 6000 cells/mm$^3$.

The inventive three-dimensional tissue culture was also successfully obtained despite varying the hydrogel (both synthetic and natural hydrogels were tested) and despite varying the medium conditions (Hamm's, DMEM and chondrogenic differentiation medium were tested, among others) and despite varying medium exchange timetables.

Finally, the three-dimensional tissue culture was successfully obtained even when using human MSCs as chondrocyte source.

Taken together, the inventive method has turned out to be extraordinarily robust for successfully obtaining the tissue culture of the present invention.

**Claims**

1. A method for manufacturing a three-dimensional tissue culture comprising chondrocytes in a biocompatible artificial matrix, the method comprising the steps of:

   - providing chondrocytes;
   - dispersing the chondrocytes in an aqueous solution, wherein the solution comprises polymerisable molecules, such that a homogenous dispersion is obtained;
   - transferring at least a part of the dispersion into a casting mould;
   - exposing the dispersion in the casting mould to conditions which allow polymerisation of the polymerisable molecules to obtain a matrix in which chondrocytes are present, wherein the matrix has a BSA diffusion coefficient of 2.5 $\times$ 10$^{-11}$ cm$^2$/s to 1 $\times$ 10$^{-6}$ cm$^2$/s at a temperature of 20°C; and
   - culturing the chondrocytes in the matrix under growth conditions, wherein a portion of the surface of the matrix is in contact with a growth medium,

   wherein said casting mould is a cell chamber of a microfluidic chip and said growth medium is brought in contact with said portion of the surface of the matrix through a medium channel of the microfluidic chip during the culturing.

2. The method of claim 1, wherein the casting mould has a bulge,

   wherein the matrix least partially extends into the bulge of the casting mould thereby forming a matrix bulge; and
   wherein at least a portion of the matrix bulge is above the level of the growth medium.

3. The method of claim 1 or 2, wherein the chondrocytes are obtained from a primary culture of cartilage, wherein said primary culture is a two-dimensional culture.

4. The method of any one of claims 1 to 3, wherein the polymerisation comprises an enzymatic polymerisation.

5. The method of any one of claims 1 to 4, wherein said growth medium comprises transforming growth factor (TGF)-beta.

6. The method of any one of claims 1 to 5, wherein said portion of the surface of the matrix is 1%-99%, preferably 5%-85%, more preferably 10%-70%, especially 30%-40% of the total surface of the matrix.

7. A three-dimensional tissue culture, comprising chondrocytes in a biocompatible artificial matrix, the culture being obtainable by the method of any one of claims 1 to 6, having at least the following layers:

   - a first layer located at or close to a surface of the matrix, wherein chondrocytes have a mean sphericity below 0.9 and are arranged in parallel to the surface along their longest dimension; and
   - a second layer at least partially covered by the first layer, wherein chondrocytes are dispersed within the matrix with a cell density of 100 to 15000 cells per mm$^3$, and wherein the mean sphericity of the chondrocytes of the second layer is higher than the mean sphericity of the chondrocytes of the first layer,

   wherein sphericity ($\Psi$) of a cell is defined as

$$\Psi = \frac{\pi^{\frac{1}{3}} \left(6V_p\right)^{\frac{2}{3}}}{A_p}$$

wherein $V_p$ is the volume of the cell and $A_p$ is the surface area of the cell.

8. The culture of claim 7, further comprising:

- a third layer at least partially covered by the second layer, wherein chondrocytes are arranged into columns extending into the matrix, wherein each column has at least two chondrocytes.

9. The culture of any one of claims 7 to 8, wherein the culture has a Shore-A hardness score of less than 90, preferably less than 85, more preferably less than 80, even more preferably less than 75, in particular less than 70.

10. The culture of any one of claims 7 to 9, wherein the culture is free of at least one the following features: tidemark, calcified cartilage and arcades of Benninghoff, optionally with subchondral bone anchorage therein; preferably free of at least two of said features, in particular free of at least three of said features.

11. The culture of any one of claims 7 to 10, wherein the matrix is at least partially composed of a biocompatible gel, preferably a hydrogel, especially a fibrin hydrogel.

12. A device comprising the three-dimensional tissue culture of any one of claims 7 to 11.

13. The device of claim 12, wherein the device is a microfluidic chip.

14. The device of claim 13, further comprising a channel for growth medium, wherein a portion of the surface of the culture is in contact with said channel, preferably wherein said portion comprises or is at least a portion of the first layer.

15. Use of the device of claim 12 or 13 as a cartilage injury model, especially as an osteoarthritis model.

**Patentansprüche**

1. Verfahren zur Herstellung einer dreidimensionalen Gewebekultur, aufweisend Chondrozyten in einer biokompatiblen künstlichen Matrix, wobei das Verfahren die folgenden Schritte aufweist:

- Bereitstellen von Chondrozyten;
- Dispergieren der Chondrozyten in einer wässrigen Lösung, wobei die Lösung polymerisierbare Moleküle aufweist, sodass eine homogene Dispersion erhalten wird;
- Überführen mindestens eines Teils der Dispersion in eine Gießform;
- Aussetzen der Dispersion in der Gießform gegenüber Bedingungen, die eine Polymerisation der polymerisierbaren Moleküle ermöglichen, um eine Matrix zu erhalten, in der Chondrozyten vorhanden sind, wobei die Matrix einen Diffusionskoeffizienten von BSA von $2{,}5 \times 10^{-11}$ cm$^2$/s bis $1 \times 10^{-6}$ cm$^2$/s bei einer Temperatur von 20 °C hat; und
- Kultivieren der Chondrozyten in der Matrix unter Wachstumsbedingungen, wobei ein Teil der Oberfläche der Matrix in Kontakt mit einem Nährmedium ist,

wobei die Gießform eine Zellkammer eines mikrofluidischen Chips ist und das Nährmedium während des Kultivierens durch einen Mediumkanal des mikrofluidischen Chips mit dem Teil der Oberfläche der Matrix in Kontakt gebracht wird.

2. Verfahren nach Anspruch 1, wobei die Gießform eine Wölbung hat,

wobei sich die Matrix zumindest teilweise in die Wölbung der Gießform erstreckt, wodurch eine Matrixwölbung gebildet wird; und
wobei mindestens ein Teil der Matrixwölbung über dem Niveau des Nährmediums liegt.

3. Verfahren nach Anspruch 1 oder 2, wobei die Chondrozyten aus einer primären Kultur aus Knorpel erhalten werden, wobei die primäre Kultur eine zweidimensionale Kultur ist.

4. Verfahren nach einem der Ansprüche 1 bis 3, wobei die Poymerisation eine enzymatische Polymerisation umfasst.

5. Verfahren nach einem der Ansprüche 1 bis 4, wobei das Nährmedium Transforming Growth Factor (TGF)-beta aufweist.

6. Verfahren nach einem der Ansprüche 1 bis 5, wobei der Teil der Oberfläche der Matrix 1 % bis 99 %, vorzugsweise 5 % bis 85 %, stärker bevorzugt 10 % bis 70 %, insbesondere 30 % bis 40 % der gesamten Oberfläche der Matrix beträgt.

7. Dreidimensionale Gewebekultur, aufweisend Chondrozyten in einer biokompatiblen künstlichen Matrix, wobei die Kultur durch das Verfahren nach einem der Ansprüche 1 bis 6 erhaltbar ist, wobei sie mindestens die folgenden Schichten hat:

- eine erste Schicht, die sich an oder in der Nähe einer Oberfläche der Matrix befindet, wobei Chondrozyten eine mittlere Sphärizität unter 0,9 haben und entlang ihrer längsten Abmessung parallel zu der Oberfläche angeordnet sind; und

- eine zweite Schicht, die mindestens teilweise von der ersten Schicht bedeckt wird, wobei Chondrozyten innerhalb der Matrix mit einer Zelldichte von 100 bis 15 000 Zellen pro mm$^3$ dispergiert sind, und wobei die mittlere Sphärizität der Chondrozyten der zweiten Schicht höher als die mittlere Sphärizität der Chondrozyten der ersten Schicht ist,

wobei die Sphärizität ($\Psi$) einer Zelle definiert ist als

$$\Psi = \frac{\pi^{\frac{1}{3}} \left(6 V_p\right)^{\frac{2}{3}}}{A_p}$$

wobei $V_p$ das Volumen der Zelle und $A_p$ die Oberfläche der Zelle ist.

8. Kultur nach Anspruch 7, ferner aufweisend:

- eine dritte Schicht, die mindestens teilweise von der zweiten Schicht bedeckt wird, wobei Chondrozyten in Säulen angeordnet sind, die sich in die Matrix erstrecken, wobei jede Säule mindestens zwei Chondrozyten hat.

9. Kultur nach einem der Ansprüche 7 bis 8, wobei die Kultur einen Shore-Härte A-Wert von weniger als 90, vorzugsweise weniger als 85, bevorzugter weniger als 80, noch bevorzugter weniger als 75, insbesondere weniger als 70 hat.

10. Kultur nach einem der Ansprüche 7 bis 9, wobei die Kultur frei von mindestens einem der folgenden Merkmale ist: Tidemark, kalzifiziertem Knorpel und Benninghoff-Arkaden, optional mit subchondraler Knochenverankerung darin; vorzugsweise frei von mindestens zwei der Merkmale, insbesondere frei von mindestens drei der Merkmale.

11. Kultur nach einem der Ansprüche 7 bis 10, wobei die Matrix mindestens teilweise aus einem biokompatiblen Gel, vorzugsweise einem Hydrogel, insbesondere einem Fibrinhydrogel, besteht.

12. Vorrichtung, welche die dreidimensionale Gewebekultur nach einem der Ansprüche 7 bis 11 aufweist.

13. Vorrichtung nach Anspruch 12, wobei die Vorrichtung ein mikrofluidischer Chip ist.

14. Vorrichtung nach Anspruch 13, die ferner einen Kanal für ein Nährmedium aufweist, wobei ein Teil der Oberfläche der Kultur in Kontakt mit dem Kanal ist, wobei der Teil vorzugsweise mindestens einen Teil der ersten Schicht aufweist oder dieser ist.

15. Verwendung der Vorrichtung nach Anspruch 12 oder 13 als Modell für Knorpelverletzung, insbesondere als Arthrosemodell.

**Revendications**

1. Procédé d'obtention d'une culture de tissu tridimensionnelle comprenant des chondrocytes dans une matrice artificielle biocompatible, le procédé comprenant les étapes suivantes :

- fournir des chondrocytes ;
- disperser les chondrocytes dans une solution aqueuse, dans laquelle la solution comprend des molécules polymérisables, de manière à obtenir une dispersion homogène ;
- transférer au moins une partie de la dispersion dans un moule de coulée ;
- exposer la dispersion dans le moule de coulée à des conditions qui permettent la polymérisation des molécules polymérisables pour obtenir une matrice dans laquelle des chondrocytes sont présents, dans laquelle la matrice a un coefficient de diffusion d'ASB de 2,5 $\times$ 10$^{-11}$ cm$^2$/s à 1 $\times$ 10$^{-6}$ cm$^2$/s à une température de 20 °C ; et
- cultiver les chondrocytes dans la matrice dans des conditions de croissance, dans laquelle une partie de la surface de la matrice est en contact avec un milieu de croissance,

dans lequel ledit moule de coulée est une chambre cellulaire d'une puce microfluidique et ledit milieu de croissance est mis en contact avec ladite partie de la surface de la matrice à travers un canal de milieu de la puce microfluidique pendant la culture.

2. Procédé de la revendication 1, dans lequel le moule de coulée présente un renflement,

dans lequel la matrice s'étend au moins partiellement dans le renflement du moule de coulée, formant ainsi un renflement de matrice ; et dans lequel au moins une partie du renflement de matrice est au-dessus du niveau du milieu de croissance.

3. Procédé de la revendication 1 ou 2, dans lequel les chondrocytes sont obtenus à partir d'une culture primaire de cartilage, dans lequel ladite culture primaire est une culture bidimensionnelle.

4. Procédé de l'une quelconque des revendications 1 à 3, dans lequel la polymérisation comprend une polymérisation enzymatique.

**5.** Procédé de l'une quelconque des revendications 1 à 4, dans lequel ledit milieu de croissance comprend un facteur de croissance transformant (TGF) bêta.

**6.** Procédé de l'une quelconque des revendications 1 à 5, dans lequel ladite partie de la surface de la matrice représente 1 % à 99 %, de préférence 5 % à 85 %, plus préférablement 10 % à 70 %, en particulier 30 % à 40 % de la surface totale de la matrice.

**7.** Culture de tissu tridimensionnelle, comprenant des chondrocytes dans une matrice artificielle biocompatible, la culture pouvant être obtenue par le procédé de l'une quelconque des revendications 1 à 6 et comprenant au moins les couches suivantes :

    - une première couche située au niveau ou à proximité d'une surface de la matrice, dans laquelle les chondrocytes ont une sphéricité moyenne inférieure à 0,9 et sont disposés parallèlement à la surface le long de leur plus longue dimension ; et
    - une deuxième couche au moins partiellement recouverte par la première couche, dans laquelle les chondrocytes sont dispersés dans la matrice avec une densité cellulaire de 100 à 15 000 cellules par mm$^3$, et dans laquelle la sphéricité moyenne des chondrocytes de la deuxième couche est supérieure à la sphéricité moyenne des chondrocytes de la première couche,

dans laquelle la sphéricité ($\psi$) d'une cellule est définie comme suit

$$\Psi = \frac{\pi^{\frac{1}{3}} \left(6 V_p\right)^{\frac{2}{3}}}{A_p}$$

où $V_p$ est le volume de la cellule et $A_p$ est l'aire de la cellule.

**8.** Culture de la revendication 7, comprenant en outre :

    - une troisième couche recouverte au moins partiellement par la deuxième couche, dans laquelle les chondrocytes sont disposés en colonnes s'étendant dans la matrice, dans laquelle chaque colonne comporte au moins deux chondrocytes.

**9.** Culture de l'une quelconque des revendications 7 et 8, dans laquelle la culture a un indice de dureté Shore A inférieur à 90, de préférence inférieur à 85, plus préférablement inférieur à 80, encore plus préférablement inférieur à 75, en particulier inférieur à 70.

**10.** Culture de l'une quelconque des revendications 7 à 9, dans laquelle la culture est dépourvue d'au moins une des caractéristiques suivantes : ligne de calcification (tidemark), cartilage calcifié et arcades de Benninghoff, comportant éventuellement un ancrage osseux sous-chondral ; de préférence dépourvue d'au moins deux desdites caractéristiques, en particulier dépourvue d'au moins trois desdites caractéristiques.

**11.** Culture de l'une quelconque des revendications 7 à 10, dans laquelle la matrice est au moins partiellement composée d'un gel biocompatible, de préférence un hydrogel, notamment un hydrogel de fibrine.

**12.** Dispositif comprenant la culture de tissu tridimensionnelle de l'une quelconque des revendications 7 à 11.

**13.** Dispositif de la revendication 12, dans lequel le dispositif est une puce microfluidique.

**14.** Dispositif de la revendication 13, comprenant en outre un canal pour le milieu de croissance, dans lequel une partie de la surface de la culture est en contact avec ledit canal, de préférence dans lequel ladite partie comprend ou constitue au moins une partie de la première couche.

**15.** Utilisation du dispositif de la revendication 12 ou 13 comme modèle de lésion cartilagineuse, en particulier comme modèle d'ostéoarthrite.

Large chip                    Small chip

**(a)**

**(b)**

**Figure 1**

Figure 2

Figure 3

**Figure 4A**

**Figure 4B**

**Comparison of cell morphology**

**Figure 5**

A               B               C

**Figure 6**

**Figure 7A**               **Figure 7B**

**Figure 8**

**Figure 9**

**Figure 10**

**Figure 11**

A

Chip design

B

0 kPa

40 kPa

C

Medium

actuator

3D-hydrogel

**Figure 12**

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010101708 A2
- US 2003040113 A1
- WO 2016174607 A1
- WO 2006017176 A2
- US 20160201037 A1

### Non-patent literature cited in the description

- **DALSTRA et al.** *Int. J. Epidemiol.,* 2005, vol. 34, 316-326
- **GELSE et al.** *Osteoarthr. Cart.,* 2012, vol. 20, 162-171
- **FELSON et al.** *Ann. Int. Med.,* 2000, vol. 133, 726-737
- **CAPITO et al.** *Osteoarthr. Cart.,* 2006, vol. 14, 1203-1213
- **SUN et al.** *Biomaterials,* 2011, vol. 32, 5581-5589
- **BACHMANN et al.** Students and Young Investigators in Regenerative Medicine Scientific Forum. Danube University Krems, 01 April 2016
- **ERTL et al.** *Europ. Pharm. Cont.,* 2009, 52-54
- **TORTELLI.** *European Cells and Materials,* 2009, vol. 17, 1-14
- **STICKER et al.** *LAB ON A CHIP,* 2015, vol. 15 (24), 4542-4554
- *Journal of Geology,* 1935, vol. 43 (3), 250-280
- **AMINI et al.** Three-dimensional in situ zonal morphology of viable growth plate chondrocytes: A confocal microscopy study. *Journal of Orthopaedic Research,* 2011, vol. 29 (5), 710-717
- **DARMANIS et al.** Static indentation test for neocartilage surface hardness in repair of periosteal articular cartilage defects. *Acta orthopaedica belgica,* 2006, vol. 72 (5), 621
- **BAE, WON C. et al.** Indentation testing of human cartilage: sensitivity to articular surface degeneration. *Arthritis & Rheumatism,* 2003, vol. 48 (12), 3382-3394
- **REDLER, IRVING et al.** The ultrastructure and biomechanical significance of the tidemark of articular cartilage. *Clinical orthopaedics and related research,* 1975, vol. 112, 357-362
- **FERGUSON et al.** Nanomechanical properties and mineral concentration in articular calcified cartilage and subchondral bone. *Journal of Anatomy,* 2003, vol. 203 (2), 191-202
- **WILSON, W. et al.** Stresses in the local collagen network of articular cartilage: a poroviscoelastic fibril-reinforced finite element study. *Journal of biomechanics,* 2004, vol. 37 (3), 357-366
- **AUGELLO ; DE BARI.** The regulation of differentiation in mesenchymal stem cells. *Human gene therapy,* 2010, vol. 21 (10), 1226-1238
- **BELLUCCI et al.** Mechanical behaviour of articular cartilage under tensile cyclic load. *Rheumatology,* 2001, vol. 40 (12), 1337-1345
- **KORHONEN, R. K. et al.** Comparison of the equilibrium response of articular cartilage in unconfined compression, confined compression and indentation. *Journal of biomechanics,* 2002, vol. 35 (7), 903-909
- **WONG et al.** Biomechanics of cartilage articulation: effects of lubrication and degeneration on shear deformation. *Arthritis & Rheumatology,* 2008, vol. 58 (7), 2065-2074
- **RIEHL et al.** *Cells,* 2012, vol. 1, 1225-1245
- **HAMILTON et al.** *Biotechnol. J.,* 2013, vol. 8, 485-495
- **KURTH et al.** *Curr. Op. Chem. Biol.,* 2012, vol. 16, 400-408
- **ROSSER, J. M. et al.** Recent Advances of Biologically Inspired 3D Microfluidic Hydrogel Cell Culture Systems. *J Cell Biol Cell Metab,* 2015, vol. 2 (005), 1-14
- **SHKILNYY, ANDRIY et al.** Diffusion of rhodamine B and bovine serum albumin in fibrin gels seeded with primary endothelial cells. *Colloids and Surfaces B: Biointerfaces,* 2012, vol. 93, 202-207
- **GOSSET, MARJOLAINE et al.** Primary culture and phenotyping of murine chondrocytes. *Nature protocols,* 2008, vol. 3 (8), 1253
- **KAMIL, S. H. et al.** Tissue engineered cartilage: utilization of autologous serum and serum-free media for chondrocyte culture. *International journal of pediatric otorhinolaryngology,* 2007, vol. 71 (1), 71-75